# EUROPEAN PATENT APPLICATION

(11) **EP 4 506 031 A1**
(43) Date of publication of application: **12.02.2025**
(21) Application number: 23854980.2
(22) Date of filing: 19.05.2023
(51) Int. Cl.: A61N 1/36, A61N 1/04, A61B 5/00, A61B 5/0531

(54) **METHOD FOR APPLYING ELECTRICAL STIMULATION TO VAGUS NERVE BY REFLECTING IMPEDANCE MEASUREMENT, AND VAGUS NERVE STIMULATION DEVICE**

(30) Priority: 17.08.2022 KR 20220102867
(71) Applicant: Neurive Co., Ltd., Juchon-myeon Gimhae-si, Gyeongsangnam-do 50969 (KR)
(72) Inventor: SONG, Jae Jun, Seoul 06501 (KR); CHOI, Hyuk, Seoul 06095 (KR); HONG, Ki Hwan, Seoul 03709 (KR); MOON, Byoung Jong, Siheung-si, Gyeonggi-do 14922 (KR)
(74) Representative: Franke, Dirk
(86) International application number: PCT/KR2023/006862
(87) International publication number: WO 2024/039007

(57) **Abstract**

The present invention relates to a method for applying electrical stimulation to the vagus nerve through an electrode in contact with the skin of a human body, the method comprising: an electrical stimulation guideline input step of inputting electrical stimulation guidelines including numerical values for electrical stimulation; an initial input value derivation step of measuring the impedance between the electrode and the skin, and using the measured impedance before applying electrical stimulation, so as to derive an input value that matches the electrical stimulation guidelines; and an electrical stimulation application step of applying electrical stimulation by using the derived initial input value. The present invention applies electrical stimulation to the electrode by reflecting the impedance between the electrode and the skin of a user, and thus can more accurately apply electrical stimulation. In addition, electrical stimulation is applied by reflecting changes in the impedance between the skin and the electrode during use, and thus accurate electrical stimulation can be continuously applied

## Description

### [Technical Field]

The present disclosure relates to a method and device for applying electrical stimulation to a vagus nerve and, in more detail, to a method and device for applying electrical stimulation to a vagus nerve through an electrode that is in contact with the skin of a human body.

### [Related Art]

The vagus nerve is one of cranial nerves and corresponds to the tenth cranial nerve. The vagus nerve is a mixed nerve coming out from the brain, distributed through the face, chest, and abdomen, and including parasympathetic nerve fibers, and takes part in controlling of parasympathetic nerves acting on the heart, lungs, alimentary canal, etc. The vagus nerve has the longest and the most complicated structure of the cranial nerves and includes all of the sensory nerve fibers and the motor nerve fibers.

Vagus nerve stimulation devices for the treatment of epilepsy and depression have reportedly received approval from the U.S. Food and Drug Administration (FDA) and these devices target the cervical branches positioned in the neck. However, such electrical stimulation of the cervical branches of the vagus nerve involves a procedure of making a direct incision in the skin, exposing the cervical branches of the vagus nerve, winding a coil, which is an electrolyte, around it, and implanting a microchip. Accordingly, it cannot be used in the realm of self-treatment by the general public.

Meanwhile, eastern medicine applies acupuncture on the ear as a method to treat diseases, the ear is an area where auricular branch of the vagus nerve is distributed, and it can be considered that stimulation of the vagus nerve is utilized for treatment.

Accordingly, devices that electrically stimulate a vagus nerve distributed in the ear have been developed for the purposes of alleviating symptoms, etc., but they are not generally used.

### [Disclosure]

### [Technical Problem]

The present disclosure has been made in an effort to solve the problems of the related art described above and an objective of the present disclosure is to provide a method and device for applying electrical stimulation to a vagus nerve, the method and device being able to more accurately apply electrical stimulation to a user.

### [Technical Solution]

In order to achieve the objectives, a method for applying electrical stimulation to a vagus nerve according to the present disclosure is a method for applying electrical stimulation to a vagus nerve through an electrode that is in contact with the skin of a human body and includes: an electrical stimulation guideline input step of inputting an electrical stimulation guideline comprising numerical values for electrical stimulation; an initial input value derivation step of measuring impedance between the electrode and the skin and driving an input value corresponding to the electrical stimulation guideline using the measured impedance before electrical stimulation is applied; and an electrical stimulation application step of applying electrical stimulation on the basis of the derived initial input value.

The electrical stimulation guideline may include a current, and voltage corresponding to the current included in the electrical stimulation guideline may be derived in the initial input value derivation step.

When the initial input value derived in the initial input value derivation step is larger than a predetermined value, the electrical stimulation application step may not be performed.

An in-stimulation adjustment step of measuring impedance between the electrode and the skin and adjusting an input value corresponding to the electrical stimulation guideline by applying newly measured impedance may be performed while the electrical stimulation application step is performed.

The electrical stimulation guideline may include a current, and voltage corresponding to the current included in the electrical stimulation guideline may be derived in the in-stimulation adjustment step.

When the initial input value derived in the in-stimulation adjustment step is larger than a predetermined value, the electrical stimulation application step may be stopped.

The in-stimulation adjustment step may be performed with a predetermined interval and an interval at which a latter in-stimulation voltage adjustment step is performed may be changed on the basis of the impedance measured in the in-stimulation adjustment step.

A device for applying electrical stimulation to a vagus nerve according to another aspect of the present disclosure includes: an electrode configured to apply electrical stimulation in contact with the skin of a human body; an input unit configured to receive an electrical stimulation guideline comprising numerical values for electrical stimulation; an initial input value deriver configured to measure impedance between the electrode and the skin and derives an input value corresponding to the electrical stimulation guideline using the measured impedance before electrical stimulation is applied; and a controller configured to perform control such that the input value derived by the initial input value deriver is applied to the electrode.

The electrical stimulation guideline may include a current, and the initial input value deriver may derive voltage corresponding to the current comprised in the electrical stimulation guideline.

When the initial input value derived by the initial input value deriver is larger than a predetermined value, the controller may not apply electrical stimulation to the electrode.

The device may further include an in-stimulation input value deriver configured to measure impedance between the electrode and the skin with electrical stimulation applied to the electrode and derive voltage corresponding to the electrical stimulation guideline by reflecting the newly measured impedance, in which the controller may apply electrical stimulation on the basis of an input value derived by the in-stimulation input value deriver.

The electrical stimulation guideline may include a current, and the in-stimulation input value deriver may derive voltage corresponding to the current included in the electrical stimulation guideline.

When the voltage derived by the in-stimulation input value deriver is larger than a predetermined value, the controller may not apply electrical stimulation to the electrode.

The in-stimulation input value deriver may measure impedance between the electrode and the skin with a predetermined interval, and an interval at which impedance between the electrode and the skin is measured later may be changed on the basis of the impedance measured by the in-stimulation input value deriver.

### [Advantageous Effects]

The present disclosure configured as described above has an effect of being able to apply more accurate electrical stimulation by applying electrical stimulation to an electrode by reflecting impedance between the electrode and the skin of a user.

Further, the present disclosure has an effect being able to continuously apply accurate electrical stimulation by applying electrical stimulation while reflecting variation of impedance between the skin of a user and an electrode.

### [Brief Description of Drawings]

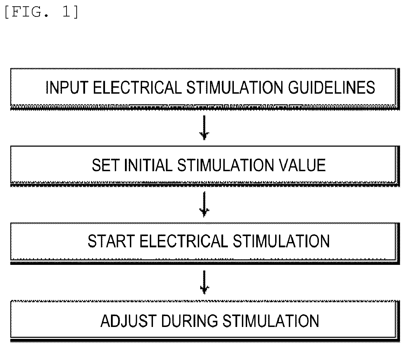

FIG. 1 is a flowchart illustrating a method for applying electrical stimulation to a vagus nerve by reflecting impedance measurement according to an embodiment of the present disclosure.

### [Mode for Invention]

Embodiments of the present disclosure are described in detail with reference to the accompanying drawings.

However, such embodiments of the present disclosure may be modified in various ways and the scope of the present disclosure is not limited only to the embodiments to be described below. The shape, sizes, etc. of elements may be exaggerated in the drawings for clearer description and elements indicated by the same reference numerals in the drawings are the same components.

Further, when an element is referred to as being "connected with" another element throughout the specification, it may be "directly connected" to the other element and may also be "electrically connected" to the other element with another element intervening therebetween. Further, unless explicitly described otherwise, "comprising" or "having" any components will be understood to imply the inclusion of other components rather than the exclusion of any other components.

Terms "first", "second", etc. are provided for discriminating one component from another component and the scope of a right is not limited to the terms. For example, the first component may be named the second component, and vice versa.

FIG. 1 is a flowchart illustrating a method for applying electrical stimulation to a vagus nerve by reflecting impedance measurement according to an embodiment of the present disclosure.

The method of this embodiment for applying electrical stimulation to a vagus nerve performs first an electrical stimulation guideline input step.

An electrical stimulation guideline includes information about electrical stimulation for stimulating a vagus nerve. An electrical stimulation guideline may be determined on the basis diagnosis and prescription by a doctor at hospital or may be derived through a separate program or system. In this case, the electrical stimulation guideline is determined in accordance with the disease or symptoms of a user and does not reflect the wearing state, shape, etc. of a device. An electrical stimulation guideline for stimulating a vagus nerve may include information such as the current, frequency, duty ratio, stimulation time of electrical stimulation. An electrical stimulation guideline including predetermined information about electrical stimulation, as described above, is input through an input unit of a device for applying electrical stimulation to a vagus nerve. As a method of inputting an electrical stimulation guideline, a user may manually input information about electrical stimulation or it may be possible to automatically receive an electrical stimulation guideline through a code (a barcode or a QR code) or an information storage device that includes information about electrical stimulation.

Next, an initial input value derivation step is performed.

An initial input value represents an electrical signal that is applied to an electrode and an electrical stimulation guideline is primarily expressed in terms of information about electricity that is applied to a human body. The initial input value means information that is applied to an electrode of a device for applying electrical stimulation. In this case, a frequency in an electrical stimulation guideline is involved with the penetration power of electrical stimulation and a current in an electrical stimulation guideline is involved with transmission of electrical transmission, which is applied to a vagus nerve, to the brain. Currently, current values of electrical stimulation for a vagus nerve are primarily studied in cases where they directly come in contact with a vagus nerve in a human body. However, since the existing devices for applying electrical stimulation adjust the voltage that is applied to an electrode, there is a problem that it is difficult to accurately apply an electrical stimulation guideline including information about a current. Accordingly, the present disclosure measures impedance between an electrode and the skin that is in contact with the electrode, derives voltage at which a current included in an electrical stimulation guideline can reach a vagus nerve by reflecting the measured impedance, and applies the voltage to the electrode, thereby being able to apply more accurate electrical stimulation to a vagus nerve. The method of measuring impedance between an electrode and the skin is not specifically limited and all of methods that can measure impedance between an electrode and the skin without interfering with the characteristics of the present disclosure can be applied. As described above, the process of deriving an initial input value corresponding to an input electrical stimulation guideline is performed by an initial input value deriver and the initial input value deriver may include an impedance meter that can measure impedance between an electrode and the skin. The impedance meter may be included in a circuit or a circuit board that applies electrical stimulation to an electrode or may be a separate impedance meter may be provided.

Further, an electrical stimulation application step of applying an initial input value derived using measured impedance to an electrode is performed.

In this case, as described above, a numerical value that is derived from an initial input value using impedance corresponds to voltage, and when voltage is larger than a predetermined value, the electrical stimulation application step may not be applied. In this case, when impedance excessively increases and voltage is increased to compensate for it, the electrical load applied to skin tissues increases, whereby injuries to the skin such as a burn may be generated. Accordingly, it may be possible to inform a user of the danger through a warning without applying electrical stimulation to an electrode. In particular, when the impedance between an electrode and the skin is excessively high, guidance may also be provided to take measures such as adding gel between the skin and an electrode. The operation of applying an initial input value derived by reflecting impedance between the skin and an electrode to an electrode is performed by a controller that controls the electricity that is applied to the electrode.

Thereafter, the present disclosure does not just maintain the initial input value as it is and performs an in-stimulation adjustment step of adjusting electrical stimulation that is applied to an electrode.

In the present disclosure, an initial input value is derived by reflecting impedance between the skin and an electrode that is measured before electrical stimulation is applied, but impedance between the skin and an electrode may change over time due to surrounding environmental factors, such as humidity, and other sudden variables. Accordingly, when an initial input value is applied as it is to an electrode, there may be a problem that inaccurate electrical stimulate may be applied to an electrode and a vagus nerve due to changed impedance. Accordingly, in an embodiment of the present disclosure, an in-stimulation adjustment step of measuring again impedance between the skin and an electrode with electrical stimulation applied to the electrode and of changing an input value that is input to the electrode using the measured impedance is performed.

Measurement of impedance with electrical stimulation applied and corresponding adjustment of the electrical stimulation may be performed in real time, but impedance between the skin and an electrode is not rapidly changed unless it is a specific case, so the measurement and adjustment may be performed with a predetermined interval. In this case, the interval between measurement of impedance and corresponding adjustment of electrical stimulation may be determined on the basis of the impedance measured before. The main issue that arises with variation of impedance is when the impedance increases significantly, and this is because when the impedance of the skin increases and voltage is increased to compensate for it, the electrical load applied to skin tissues increases, whereby injuries to the skin such as a burn may be generated. However, when voltage is not sufficiently increased as much as increased impedance, the current between electrodes decreases, so there may be a problem that the effect of stimulation of a vagus nerve is deteriorated. Accordingly, the higher the impedance, the more frequently impedance measurement and input value adjustment may be performed, and the lower the impedance, the larger the interval between impedance measurement and input value adjustment. Even in the process of performing adjustment during stimulation, when voltage is larger than a predetermined value, it may be possible to stop the electrical stimulation application step and inform a user of the situation through a warning, etc. The process of measuring impedance and deriving a new input value while electrical stimulation is applied is performed by an in-stimulation input value deriver, and the in-stimulation input value deriver can use all of some of components with the initial input value deriver. The input value derived by the in-stimulation input value deriver is applied to the electrode through the controller.

Exemplary embodiments of the present disclosure were described above, but it would be understood by those skilled in the art that these embodiments described above are only examples of the spirit of the present disclosure and the present disclosure may be changed in various ways without departing from the spirit of the present disclosure. Accordingly, the protective range of the present disclosure should be construed on the basis of claims rather than specific embodiments and all of spirits within the equivalent range should be construed as being included in the range of right of the present disclosure.

## Claims

1. A method for applying electrical stimulation to a vagus nerve that is a method for applying electrical stimulation to a vagus nerve through an electrode that is in contact with the skin of a human body, the method comprising:
an electrical stimulation guideline input step of inputting an electrical stimulation guideline comprising numerical values for electrical stimulation;
an initial input value derivation step of measuring impedance between the electrode and the skin and deriving an input value corresponding to the electrical stimulation guideline using the measured impedance before electrical stimulation is applied; and
an electrical stimulation application step of applying electrical stimulation on the basis of the derived initial input value.

2. The method of claim 1, wherein the electrical stimulation guideline comprises a current, and voltage corresponding to the current included in the electrical stimulation guideline is derived in the initial input value derivation step.

3. The method of claim 2, wherein when the initial input value derived in the initial input value derivation step is larger than a predetermined value, the electrical stimulation application step is not performed.

4. The method of claim 1, wherein an in-stimulation adjustment step of measuring impedance between the electrode and the skin and adjusting an input value corresponding to the electrical stimulation guideline by applying newly measured impedance is performed while the electrical stimulation application step is performed.

5. The method of claim 4, wherein the electrical stimulation guideline comprises a current, and voltage corresponding to the current comprised in the electrical stimulation guideline is derived in the in-stimulation adjustment step.

6. The method of claim 5, wherein when the initial input value derived in the in-stimulation adjustment step is larger than a predetermined value, the electrical stimulation application step is stopped.

7. The method of claim 4, wherein the in-stimulation adjustment step is performed with a predetermined interval.

8. The method of claim 7, wherein an interval at which a latter in-stimulation voltage adjustment step is performed is changed on the basis of the impedance measured in the in-stimulation adjustment step.

9. A device for applying electrical stimulation to a vagus nerve, the device comprising:
an electrode configured to apply electrical stimulation in contact with the skin of a human body;
an input unit configured to receive an electrical stimulation guideline comprising numerical values for electrical stimulation;
an initial input value deriver configured to measure impedance between the electrode and the skin and derives an input value corresponding to the electrical stimulation guideline using the measured impedance before electrical stimulation is applied; and
a controller configured to perform control such that the input value derived by the initial input value deriver is applied to the electrode.

10. The device of claim 9, wherein the electrical stimulation guideline comprises a current, and the initial input value deriver derives voltage corresponding to the current comprised in the electrical stimulation guideline.

11. The device of claim 10, wherein when the initial input value derived by the initial input value deriver is larger than a predetermined value, the controller does not apply electrical stimulation to the electrode.

12. The device of claim 9, further comprising an in-stimulation input value deriver configured to measure impedance between the electrode and the skin with electrical stimulation applied to the electrode and derive voltage corresponding to the electrical stimulation guideline by reflecting the newly measured impedance,
wherein the controller can apply electrical stimulation on the basis of an input value derived by the in-stimulation input value deriver.

13. The device of claim 12, wherein the electrical stimulation guideline comprises a current, and the in-stimulation input value deriver derives voltage corresponding to the current comprised in the electrical stimulation guideline.

14. The device of claim 13, wherein when the voltage derived by the in-stimulation input value deriver is larger than a predetermined value, the controller does not apply electrical stimulation to the electrode.

15. The device of claim 12, wherein the in-stimulation input value deriver measures impedance between the electrode and the skin with a predetermined interval.

16. The device of claim 15, wherein an interval at which impedance between the electrode and the skin is measured later is changed on the basis of the impedance measured by the in-stimulation input value deriver.
